Europäisches Patentamt

European Patent Office

Office européen des brevets

(19)

(11) Publication number: **0 230 343**
A2

(12) **EUROPEAN PATENT APPLICATION**

(21) Application number: 87300029.3

(22) Date of filing: **06.01.87**

(51) Int. Cl.⁴: **C 12 Q 1/26**, C 12 Q 1/48

(30) Priority: **06.01.86 US 816574**

(43) Date of publication of application: **29.07.87**
**Bulletin 87/31**

(84) Designated Contracting States: **AT BE CH DE ES FR GB GR IT LI LU NL SE**

(71) Applicant: **Isolab, Inc., 5552 Wooster Road, Barberton Ohio 44203 (US)**

(72) Inventor: **Rosenthal, Murray A., 530 Clearbrook Drive, Akron Ohio 44313 (US)**

(74) Representative: **Nash, Keith Wilfrid, KEITH W. NASH & Co. Pearl Assurance House 90-92 Regent Street, Cambridge CB2 1DP (GB)**

(54) **Method of determining the amount of phosphatidylglycerol in amniotic fluids as a diagnostic indicator.**

(57) Methods and tests to determine the concentration of phosphatidylglycerol (PG) in amniotic fluids to aid in the diagnostic indication of fetal lung maturity are described.

## METHOD OF DETERMINING THE AMOUNT OF PHOSPHATIDYLGLYCEROL IN AMNIOTIC FLUIDS AS A DIAGNOSTIC INDICATOR

### TECHNICAL FIELD

This invention relates to a method for determining a numerical concentration of phosphatidylglycerol (PG) in fluids. More particularly, this invention relates to a method of determining the amount of phosphatidylglycerol (PG) in amniotic fluids as a diagnostic indicator of fetal lung maturity (FLM).

### BACKGROUND ART

Prematurity of the infant remains a principal cause of perinatal death. In 50 to 70 percent of premature neonatal deaths, hyaline membrane disease is the primary cause. Hyaline membrane disease is caused by the lack of pulmonary surfactant. In 1971, Gluck and Associates, Am. J. Obstet. Gynecol. 109:440-445 provided a means to analyze amniotic fluid for fetal pulmonary surfactant when they described the lecithin/sphingomyelin (L/S) ratio test using thin layer chromatography (TLC) techniques. This test remains the standard for pulmonary maturity testing.

The thin layer chromatographic test is costly, time-consuming, requires highly skilled technicians and rigorous quality control. It further has numerous technical pitfalls which, in a number of cases, renders the L/S ratio unreliable; for instance, where pregnancies are complicated by diabetes mellitus, hypertension or premature rupture of membranes. Thus, a number of laboratories report infants from diabetic mothers develop respiratory distress syndrome (RDS) despite a mature L/S ratio, which indicates the infant should have no breathing difficulty.

Other tests for measuring lung maturity include the bubble or shake test and fluorescence polarization measurements, but these tests sometime give false readings.

Hallman et al, Pediatr. Res. 9:396-402 (1975) has shown that PG is absent from lung aspirates of infants with RDS and that PG appears during their recovery. Therefore, many researchers believe phosphatidylglycerol level is an important indicator of fetal lung maturity (FLM). A number of chromatographic (TLC) methods have been used to measure the PG in amniotic fluids but these methods have the defects previously enumerated therefor.

Lecithin and PG quantitations have been done enzymatically (Clin. Chem. 25: 103-107; Microchrom. J. 24: 239-258 and Am. J. Obstet. Gynecol. 145: 474-480). However, extraction with organic solvents was used in most cases. Extraction is time-consuming and messy. Hazardous solvents are used, and recoveries of phospholipids are often less than 100 percent. Also, residual amounts of organic solvent, present after drying the extract, can inactivate enzymes. Anaokar et al. Clin. Chem. 25: 103-107 and Artiss et al. Microchrom. J. 25: 153-168 and 24: 239-258 depicted an enzymatic procedure for lecithin without extraction of amniotic fluid. Amniotic fluid samples were either clarified by centrifugation or not clarified at all. Centrifugation causes a loss of phospholipid material before analysis. Unclarified samples cause altered or unstable absorbance readings. Anaokar et al used a post enzymatic extraction step to avoid turbidity problems, but this reintroduces many of the aforementioned disadvantages.

An agglutination test for amniotic fluid PG has also been introduced. It is rapid and is specific for PG. However, it is only semi-quantitative, is costly, has reagents which may be unstable (to time, temperature), and is technique dependent.

The presence or absence of phosphatidylglycerol is a predictor of whether an infant has substantial risk of RDS. Thus, a quantitative non-chromatographic method for determining PG would have advantages over currently popular TLC methods.

## DISCLOSURE OF INVENTION

An aspect of this invention is to provide methods to determine the concentration of PG in amniotic fluid.

A quantity of amniotic fluid obtained by trans-abdominal amniocentesis is the sample of choice. However, vaginally obtained specimens are also acceptable. The sample of amniotic fluid must then be treated in order to solubilize the PG contained in liposomal or membranous aggregates, i.e., to form a solution, and to clarify the sample of particulate and/or flocculent material preferably prior to subsequent enzymatic analysis.

The amniotic fluid preferably is freed from aggregates and solubilized by treatment of the sample with a surfactant such as an aqueous solution of Triton X-100 to give a solution or dispersion of PG. Other methods such as ultrasonic disruption, mechanical disruption, freeze/thaw cycle(s), sudden decompression, and vortex mixing may be used to free PG from the aggregates to yield a solution.

After the PG is solubilized, particulate and/or flocculent material is removed by centrifugation or filtration. It is important, when using filtration, to ensure the filtering media does not absorb PG. Alternatively, the clarification step may be done during or after enzymatic analysis or not done at all. A combination of filtration and centrifugation can also be used.

Thereafter, the amniotic fluid sample is mixed with an enzyme or enzymes that destroys free glycerol and incubated for a period of time to remove any glycerol present. Coenzymes, activators, enhancers, stabilizers, solubilizers, antimicrobials, etc., may also be present in the mixture but generally should not interfere with the test.

The amniotic fluid sample, free of endogenous glycerol, is treated with another enzyme or enzymes sometimes hereinafter called glycerol liberating enzymes, to liberate glycerol from the phosphatidylglycerol. The liberated glycerol is tested in the presence of a color indicator, a chromogen, to develop a color and the color is determined and compared with a standard to arrive at the phosphatidylglycerol concentration in the amniotic fluid.

## BRIEF DESCRIPTION OF THE DRAWINGS

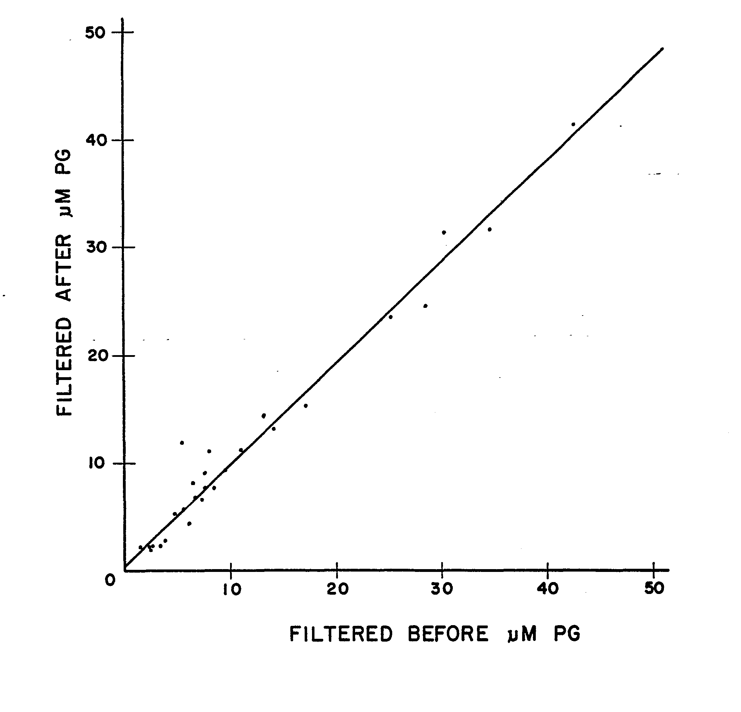

The drawing is a graph showing filtering before enzymatic incubation has no effect on the accuracy of PG analysis.

## BEST MODE FOR CARRYING OUT THE INVENTION

Practice of the method according to this invention requires collection of an amniotic fluid sample from a pregnant female. Amniotic fluid can be obtained either by trans-abdominal amniocentesis or collection of a vaginal specimen by conventional techniques and well known procedures.

The amniotic fluid is then treated to prepare a test sample by a procedure to free PG from liposomal or membranous aggregates and to solubilize it. A preferred procedure involves mixing the amniotic fluid with a surfactant. The mixing can be done manually, on a vortex mixer, or other mixers. The period of time needed to treat the sample (both mixing and sitting time) is dependent on the condition of the sample, the efficiency of surfactant used, and the efficiency of the mixing process. The surfactant may be any detergent or emulsifier, or combinations or dilutions thereof, capable of solubilizing PG. McCutcheon's Emulsifiers and Detergents, North American Edition, 1983, MC Publishing Co., Glen Rock, New Jersey 07452 lists numerous surfactants, any of which could be suitable for this purpose, provided they do not interfere with the enzymatic determination. The criterion of choice is limited only by the efficiency of the solubilization process and the non-interference with the subsequent assay. The more efficient the surfactant, the less of it (by volume) that has to be used, which in turn increases the sensitivity of the detection by reducing the dilution of the sample.

Another procedure which can be used to prepare a test sample involves physical disruption of the PG-containing aggregates. This can be done in the presence or absence of a surfactant. Physical disruption can be accomplished by well known methods such as freeze-thaw cycles, ultrasonic devices, and sudden decompression, as well as prolonged and/or violent mixing.

Optionally, the test sample may be clarified by filtration or centrifugation. Clarification results in a test sample suitable for spectrophotometric analysis. Clarification need not be done if the sample is free of particulate or flocculent matter or if a non-spectrophotometric detection method is used. Although clarification may be done before the solubilization step, loss of PG analyate can occur. Alternatively, the clarification step may be done during or after enzymatic analysis. As indicated above, clarification can be accomplished by either centrifugation or filtration or a combination of the two procedures. Also the sample may be filtered and centrifuged at the same time. The choice of filtration apparatus and filter media is limited only by the desired clarity, speed of filtration, non-absorption of PG, and non-interference in the subsequent assay. Alternatively, the sample may be ultrafiltered to remove both particulates and proteins such as hemoglobin, which may interfere in the subsequent assay.

A predetermined quantity of the prepared test sample is mixed with a predetermined quantity of reagent(s) containing the desired enzymes and is allowed to incubate for a period of time. These enzymes function so as to remove from said mixture substance(s) which would interfere with subsequent analysis. The primary interfering substance is glycerol. Secondary interferences include substrate(s) of the subsequent detection reactions (i.e., glycerol-3-phosphate and peroxide). These enzymes also function, during the subsequent color formation step, to convert glycerol derived from PG to color. The following enzymatic system is preferred (E.C. number is designated in parenthesis):

glycerol kinase (2.7.1.30)
glycerophosphate oxidase (no E.C. #assigned)
peroxidase (1.11.1.7)

Glycerol is converted to glycerol-phosphate by glycerol kinase (GK) in the presence of ATP. Glycerol-phosphate is converted to dihydroxyacetone phosphate and hydrogen peroxide by glycerophosphate oxidase (GPO) in the presence of oxygen. Peroxide is converted to water and oxygen by peroxidase (POD).

The enzyme-containing reagent may also contain water, buffers, salts, coenzymes, antimicrobial agents, solubilizers, activators, enhancers, stabilizers, and/or redox couplers. Redox couples are part of the peroxidase indicator system. These couplers function to oxidatively couple peroxidase with a colorimetric indicator compound which is added after the interference removal step. During the interference removal step, couplers also function to eliminate peroxide using a non-color forming reaction. Thus any peroxide which is present or is generated during the interference removal step is eliminated and does not interfere with the subsequent colorimetric indicator system. The enzymes are incubated for a predetermined time at a predetermined temperature, until said interfering substances are reacted or removed completely (or nearly so), usually temperatures of 20° to 50°C for 5 to 30 minutes is adequate.

Two identical portions of the above incubated mixtures are obtained either by treating two portions of test sample with identical predetermined amounts of enzymes or treating one portion of test sample with a predetermined amount of enzymes and then splitting it into two equal aliquots. One portion or aliquot is designated as the blank aliquot and the other one as the sample aliquot.

An enzymatic indicator solution is prepared to contain reagent(s), which detect enzymatic activity of one or more of the reactions of the enzymatic system. The preferred type of indicator (colorimetric) should remain colorless in the absence of enzymatic activity, and should produce color, which can be measured spectrophotometrically or estimated visually, in the presence of activity. The indicator may be a single compound or a combination of compounds. If two or more compounds are used, one or more of them may be added to the original glycerol enzyme reagent (or directly to the sample) prior to the interference removal step, as long as little or no color formation occurs during the interference removal step. Enzymatic activity measurements can be determined also by the following well known methods: (1) kinetic, (2) radiochemical, (3) fluorometric, (4) chemiluminescent, (5) turbidimetric, and (6) immunologic.

The enzymatic indicator solution may also include the well known buffers, salts, coenzymes, antimicrobial agents, solubilizers, activators, enhancers, and/or stabilizers.

The preferred glycerophospholipase enzyme is phospholipase D (E.C. 3.1.4.4.). This enzyme functions to generate a substrate (glycerol) from PG for the enzymes which were present during the interference removal step. These enzymes are added to the sample aliquot (not the blank aliquot) either separately or together with the enzymatic indicator solution.

Next, a predetermined quantity of enzymatic indicator solution is added to each blank aliquot and a predetermined quantity of enzymatic indicator solution and glycerophospholipase enzyme (either separately or together) is added to each sample aliquot. All aliquots are then mixed and incubated for a predetermined time at a predetermined temperature, as discussed above, until a point where enzymatic activity can be measured as indicated above.

As is appropriate in the practice of clinical chemistry techniques, controls and/or standards may also be used in the practice of this invention. They are assayed alongside samples in the same manner as the samples. Controls and standards are used to assure validity of the test, assure the test is functioning properly, and to evaluate sample data. Controls and/or standards consist of a known amount of analyate contained in either a biological matrix and/or solution. They may also contain the well known buffers, salts, antimicrobial agents, solubilizers, stabilizers, and/or contaminants, i.e., glycerol. Assayed values for controls and/or standards in this enzymatic PG assay can be compared to those obtained on samples. A numeric concentration of PG in samples can then be calculated or a qualitative assessment of FLM can be made.

It is highly desirable that the various clinical laboratories have uniform standards for test purposes to facilitate cross checking and to optimize accuracy. Therefore, the reagents for performing the test method of this invention are supplied in kit form. A representative kit has the following reagents:

(a) a reagent containing at least one surfactant capable of effecting dispersion or solution of any PG present in an amniotic fluid so said dispersed or solubilized PG is not retained on the filter medium as the fluid passes through it,

(b)   a reagent capable of removing, destroying or coupling any endogenous glycerol present in the fluid,

(c)   a reagent capable of liberating glycerol from the PG, and

(d)   at least one standard containing PG.

Optionally, a filtering device may be included.

A preferred kit contains sufficient reagents, contained usually in tubes, vials or similarly closed containers, to assay at least one blank and one sample. Thus, this kit has Reagent 1 composed of an aqueous surfactant solution, viz., Triton X-100 in 2 to 10 and preferably about 3 to 6 percent by weight. Reagent 2 is a mixture of dried glycerol enzymes, such as GPO, GK or peroxidase, which can be reconstituted by addition of quantity of glycerol enzyme reconstitution reagent. Reagent 3 is a glycerol enzyme reconstitution reagent containing a redox coupler. Reagent 4 is the enzyme phospholipase D (PL-D). Reagent 5 is a color reagent which reacts with peroxide to develop a color. Reagent 6 is the optional filter columns or devices, and Reagent 7 contains comparison standards, preferably 0, 2.5 and 5.0 $\mu$M of standard PG.

The following examples will further illustrate and exemplify the practice of the invention by persons skilled in the art of clinical chemistry.

### Example 1

A portion of a well mixed sample of amniotic fluid from a pregnant female was treated with a solution containing 50 g/L Triton X-100 (Rohm & Haas), a surfactant classified as an alkylethoxy-polyethoxy ethanol, in the ratio of 10 parts AF to 1 part TX-100 solution. It was then mixed on a vortex mixer for 15 seconds, allowed to stand 5 minutes, then mixed again. The test sample was then filtered using glass fiber paper. Two 500 $\mu$L aliquots (a blank and a sample) of the prepared test sample were then placed in test tubes. Two additional test sets of 500 $\mu$L aliquots of each of the following standards were also placed in other test tubes as follows: 0, 2.5 and 5.0 $\mu$M PG contained in 5 g/L TX-100 in aqueous solution. Also two 500 $\mu$L aliquots of a 500 $\mu$M glycerol solution were also placed in the test tubes of two additional test sets. Next 500 $\mu$L of glycerol enzyme solution was added to all tubes. The glycerol enzyme solution used contained:

| | | |
|---|---|---|
| 5 | g | TX-100 |
| 18.2 | g | Tris, the abbreviation for tris(hydroxymethyl) aminoethane |
| 2.04 | g | $CaCl_2 \cdot 2\ H_2O$ |
| 1.42 | g | $MgCl_2 \cdot 6\ H_2O$ |
| 2.24 | g | MEHA, the abbreviation for 2-(N-ethyl-m-toludino)-ethanol |
| 1.11 | g | $ATP_3 \cdot H_2O$, the abbreviation for adenosine triphosphate |
| 9.33 | KU | GPO (enzyme from _Aerococcus viridans_) |
| 17.6 | KU | POD (enzyme from horseradish) |
| 1 | KU | GK (enzyme from _Streptomyces chromofuscus_ |

and had been adjusted to pH 7.6 with concentrated HCl and diluted to 1 liter with distilled water.

All the tubes were mixed and incubated at 37°C for 12 minutes. Next 50 $\mu$L of peroxidase indicator solution was added to each blank tube and 50 $\mu$L of phospholipase D/indicator solution was added to each sample tube. These solutions contained:

Peroxidase indicator solution:

| | |
|---|---|
| 18.2 g | Tris |
| 2.04 g | $CaCl_2 \cdot 2\ H_2O$ |
| 4.08 g | 4-aminoantipyrine (4-AAP) |

and had been adjusted to pH 7.6 with concentrated HCl and diluted to 1 liter with distilled water. The phospholipase D indicator solution also contained 133.4 KU/L PL-D from <u>Streptomyces chromofuscus</u>. All tubes were mixed and incubated for 12 additional minutes at 37°C. The absorbance of each tube was then measured in a spectrophotometer at 550 nm. The resultant absorbance measurements were:

|     |                     | A         | B          |       |          |
| --- | ------------------- | --------- | ---------- | ----- | -------- |
| No. | Sample              | Blank Abs | Sample Abs | B-A   | $\mu$M PG |
| 1   | AF                  | .039      | .102       | .063  | 2.39     |
| 2   | 0 $\mu$M PG std     | .021      | .069       | .048  | -0.17    |
| 3   | 2.5 $\mu$M PG std   | .019      | .084       | .065  | 2.72     |
| 4   | 5.0 $\mu$M PG std   | .021      | .099       | .078  | 4.89     |
| 5   | 500 $\mu$M glycerol | .023      | .072       | .049  | 0.06     |

The $\mu$M PG concentration was determined by linear regression analysis using the three standards. Glycerol did not interfere with the assay.

### Example 2

The effect of filtering on the recovery of the PG from AF was examined. Twenty-seven amniotic fluid samples were assayed as in Example 1 using two test conditions. In the control condition, filtering was done as in Example 1. In the experimental condition, filtering was not done until after the completion of the second incubation step, just before spectrophotometric quantitation. Results are shown on the figure of the drawing, where the data obtained by adding PG to an amniotic fluid before or after filtration yields essentially a straight line.

When the data is treated by linear regression analysis (n = 27) the correlation coefficient is 0.992 and the regression line equation is y = 0.9359 X + 0.497. At points below 15 $\mu$M PG (n = 21), the correlation coefficient is 0.954 and the regression line equation is y = 1.001 X + 0.149. This demonstrates that this filtering procedure does not remove PG from the test sample, when the surfactant is present.

## Example 3

Aliquots of a well mixed sample of AF were treated by each of the following conditions:

| Aliquot | Treatment Condition |
|---|---|
| A | Filtered and diluted by 10% with 50 g/L aqueous TX-100 solution |
| B | Centrifuged at 1000 X g for 10 minutes and diluted by 10% with 50 g/L aqueous TX-solution |
| C | Mixed with 50 g/L aqueous TX-100 solution and filtered (as in Example 1) |
| D | Mixed with 50 g/L aqueous TX-100 solution and centrifuged at 1000 X g for 10 minutes |
| E | Mixed with 50 g/L aqueous TX-114 solution and filtered (as in Example 1) |
| F | Mixed with 50 g/L Pluronic L-35* and filtered (as in Example 1) |

*A surfactant from BASF Wyandotte

A PG assay was performed on the above-treated samples using the procedure in Example 1. Results:

| ALIQUOT | $\mu$ M PG |
|---------|------------|
| A | 6.45 |
| B | 6.99 |
| C | 8.51 |
| D | 8.21 |
| E | 8.36 |
| F | 8.21 |

This demonstrates that a surfactant treatment prior to clarification results in improved recovery of PG from AF.  It also shows that other surfactants can be substituted for Triton X-100.

## Example 4

The experiment in Example 1 was repeated using different reagents which indicate peroxidase activity.  Experimental protocols were identical to Example 1 except for the following substitutions:

Trial A   Sodium 2-hydroxy-3,5-dichlorobenzene sulfonate (HDCBS) was substituted for MEHA at the same molar concentration (12.5 mM). Absorbance was read at 510 nm.

Trial B   3-hydroxy-2,4,6-tribromobenzoic acid was substituted for MEHA at the same molar concentration (12.5 nM).  Absorbance was read at 513 nm.

Trial C   3-methyl-2-benzothiazolone-hydrazone hydrochloride was substituted for 4-AAP at the same molar concentration (20 mM). Absorbance was read at 590 nm.

Absorbance measurements are tabulated hereinafter, along with those from Example 1.

| Example | Sample | A Blank Abs | B Sample Abs | B-A | Difference 0 and 5 $\mu$M Std |
|---|---|---|---|---|---|
| 1 | 0 $\mu$M PG Std | .021 | .069 | .048 | |
| 1 | 5 $\mu$M PG Std | .021 | .099 | .078 | .030 |
| 1 | 500 $\mu$M glycerol | .023 | .072 | .049 | |
| 4A | 0 $\mu$M PG Std | .038 | .080 | .042 | |
| 4A | 5 $\mu$M PG Std | .037 | .113 | .076 | .034 |
| 4A | 500 $\mu$M glycerol | .078 | .111 | .033 | |
| 4B | 0 $\mu$M PG Std | .082 | .116 | .034 | |
| 4B | 5 $\mu$M glycerol | .082 | .137 | .055 | .021 |
| 4B | 500 $\mu$M glycerol | .101 | .134 | .033 | |
| 4C | 0 $\mu$M PG Std | .140 | .183 | .043 | |
| 4C | 5 $\mu$M PG Std | .142 | .249 | .107 | .064 |
| 4C | 500 $\mu$M glycerol | .133 | .157 | .024 | |

This demonstrates that other couplers and color indicators can be used.

## Example 5

The experiment in Example 4, Trial A, was repeated except that the HDCBS was included in the color reagent (12.5 mM) and was not included in the glycerol enzymes reagent. Also 50 $\mu$M glycerol was added to the standards in order to simulate endogenous amniotic fluid glycerol. Absorbance measurements were:

| Sample | Absorbance | | |
| | A Blank | B Sample | BA Difference |
|---|---|---|---|
| 0 $\mu$M PG, 50 $\mu$M glycerol | .216 | .248 | .032 |
| 5 $\mu$M PG, 50 $\mu$M glycerol | .219 | .248 | .029 |
| 500 $\mu$M glycerol | >3.0 | >3.0 | --- |

This demonstrates that a coupler (HDCBS) must be present during the preincubation step in order to destroy products produced or present in the interference removal step. If not, any endogenous glycerol originally present will result in color formation upon addition of the color reagent.

### Example 6

The experiment in Example 1 was repeated with the following changes: 1200 $\mu$L of sample was added to one test tube. 1200 $\mu$L of the following standards were also placed in the test tubes: 0, 2.5, 5.0 $\mu$M PG contained in 5 g/L TX-100 solution. Also 1200 $\mu$L of a 500 $\mu$M glycerol solution was also placed in a test tube. Next 1200 $\mu$L of glycerol enzyme solution was added to each tube. The tubes were mixed and incubated at 37°C for 12 minutes. Two 500 $\mu$L portions (a blank and a sample aliquot) were removed from each tube and placed in separate tubes. The assay was continued as in Example 1. The resultant absorbance measurements were:

|  |  | Absorbance | | | |
| --- | --- | --- | --- | --- | --- |
|  |  | A | B | Difference | $\mu$M PG |
| No. | Sample | Blank Abs | Sample Abs | B–A | Found |
| 1 | AF | .061 | .101 | .040 | 3.37 |
| 2 | 0 $\mu$M PG Std | .023 | .049 | .026 | 0.04 |
| 3 | 2.5 $\mu$M PG Std | .024 | .060 | .034 | 2.42 |
| 4 | 5.0 $\mu$M PG Std | .026 | .073 | .047 | 5.04 |
| 5 | 500 $\mu$M glycerol | .029 | .054 | .025 | −0.20 |

This demonstrates that the initial incubation can be performed in a single tube, then split into sample and blank tubes and assayed as in Example 1.

While in accordance with the patent statutes only the best mode and preferred embodiment of the invention has been illustrated and described in detail, it is to be understood that the invention is not limited thereto or thereby, but that the scope of the invention is defined by the appended claims.

WHAT IS CLAIMED IS:

1. A method of determining phosphatidylglycerol, PG, concentration as a diagnostic indicator of fetal lung maturity comprising:

treating an amniotic fluid sample to form a dispersion or solution of said PG;

removing endogenous glycerol from said solution; and

treating the solution to liberate glycerol from PG, and determining the concentration of liberated glycerol, this concentration being indicative of the PG concentration in said amniotic fluid.

2. The method of Claim 1 wherein the amniotic fluid is treated with a surfactant, or by physical disruption, to yield a solution, having said PG solubilized therein, and the sample is treated by filtration or centrifugation, or a combination thereof, subsequent to forming a solution of PG, and wherein endogenous glycerol is removed from said solution by treatment with at least one enzyme.

3. The method of Claim 2 wherein at least one enzyme generates hydrogen peroxide, which is destroyed by at least one of the following agents: enzymes, chemical reductants, or redox couplers, wherein glycerol kinase and glycerophosphate oxidase are used to generate hydrogen peroxide, and wherein peroxidase is used to destroy hydrogen peroxide.

4. The method according to Claim 1 wherein the treatment to liberate glycerol from PG is with a glycerol liberating enzyme, and wherein phospholipase D is used as the glycerol liberating enzyme, and wherein the concentration of liberated glycerol is determined with at least one enzyme, and wherein the concentration of liberated glycerol is determined with at least one enzyme, still present, which removed endogenous glycerol from solution, and wherein at least one enzyme generates hydrogen peroxide, the concentration of which is measured using peroxidase.

5. The method according to Claim 1 wherein the concentration of liberated glycerol is determined by developing color with a color indicator, and reading the color developed, wherein hydrogen peroxide concentration is measured by using a color indicator to measure peroxidase activity, wherein peroxidase activity is measured by a color indicator and a redox coupler, wherein 2-(N-ethyl-meta-toluidino)-ethanol is used as the redox coupler, wherein 2-hydroxy-3,5-dichlorobenzene sulfonate is used as the redox coupler, and wherein 4-aminoanti-pyrene is used as the color indicator.

6. The method according to Claim 1 wherein the concentration of PG in an amniotic fluid sample is determined by comparing the color developed in said sample with the color developed in a standard, which contains PG, and wherein the standard contains both PG and glycerol.

7. A kit for assaying the PG content of an amniotic fluid sample comprising as components (1) a reagent containing at least one surfactant for treating said fluid to form a solution of said PG, (2) a reagent capable of removing endogenous glycerol from said solution, (3) a reagent capable of liberating glycerol from PG, and (4) at least one standard containing PG.

8.  The kit as claimed in Claim 7 which includes a filtering device for clarifying said solution of PG.

9.  The kit as claimed in Claim 7 wherein at least one enzyme comprises the reagent capable of removing endogenous glycerol from solution and at least one enzyme comprises the reagent capable of liberating glycerol from PG.'

10.  The kit as claimed in Claim 7 wherein a redox coupler is included in the reagent capable of removing glycerol from solution and a color indicator is included in the reagent capable of liberating glycerol from PG.

1/1